# EUROPÄISCHE PATENTSCHRIFT

(11) **EP 1 596 709 B1**
(45) Veröffentlichungstag und Bekanntmachung des Hinweises auf die Patenterteilung: **26.08.2009**
(21) Anmeldenummer: 03789375.7
(22) Anmeldetag: 20.12.2003
(51) Int. Cl.: A61B 3/00, A61F 9/01, A61B 3/135

(54) **ANORDNUNG ZUR BILDFELDVERBESSERUNG BEI OPHTHALMOLOGISCHEN GERÄTEN**
ARRANGEMENT FOR IMPROVING THE IMAGE FIELD IN OPHTHALMOLOGICAL APPLIANCES
ENSEMBLE PERMETTANT D'AMELIORER LE CHAMP D'IMAGE SUR DES APPAREILS OPHTALMOLOGIQUES

(30) Priorität: 24.02.2003 DE 10307741
(43) Veröffentlichungstag der Anmeldung: 23.11.2005
(73) Patentinhaber: Carl Zeiss Meditec AG, 07745 Jena (DE)
(72) Erfinder: KOSCHMIEDER, Ingo, 07743 Jena (DE); DOBSCHAL, Hans-Jürgen, 99510 Kleinromstedt (DE)
(74) Vertreter: Beck, Bernard
(86) Internationale Anmeldenummer: PCT/EP2003/014689
(87) Internationale Veröffentlichungsnummer: WO 2004/073511

(56) Entgegenhaltungen:
- EP-A- 0 331 469
- WO-A-02/45578
- DE-A- 19 713 138
- US-A- 5 479 221
- US-A- 5 571 107
- US-A- 5 673 096
- US-A1- 2002 111 606

## Beschreibung

Die vorliegende Erfindung betrifft eine Anordnung mit der das Bildfeld der Beleuchtungs- bzw. Bestrahlungskomponenten von ophthalmologischen Öiagnose- und Therapiegeräten verbessert wird. Die Anordnung ist insbesondere für ophthalmologische Geräte geeignet, bei denen eine gleichbleibend hohe Abbildungsqualität über breite Bereiche des Auges von Interesse ist.

Dies ist beispielsweise bei Spaltlampen der Fall. Mittels Spaltbildprojektion wird in dem zu untersuchenden Inneren des Auges ein Lichtschnitt erzeugt. Um eine exakte Auswertung der so erzeugten Schnittbilder gewährleisten zu können, ist eine scharfe Abbildung des Spaltbildes sowohl an der Sehachse als auch in den Randbereichen des Auges erforderlich.

Die vorliegende Anordnung zur Bildfeldverbesserung ist prinzipiell überall dort einsetzbar, wo eine gleichbleibend hohe Abbildungsqualität über einen breiten Bereich gewährleistet werden soll. Anwendungen sind neben der Ophthalmologie beispielsweise die Lasermedizin, die refraktive Chirurgie, und die Bestrahlung einer mittels Licht veränderbaren optischen Linse, gemäß den Patentschriften WO 00/41650 und WO 01/71411.

Bei diese Art von Linsen, die aus einer Matrix verschiedener Kunststoffe bestehen, werden durch Bestrahlung Polymerisationsvorgänge ausgelöst, die eine Veränderung des Brechungsindexes oder der Form der Linse zur Folge haben. Intraokularlinsen (IOL) dieser Art können nach der Implantation durch gezielte Bestrahlung so verändert werden, dass dadurch ein fehlerreduziertes Sehen ermöglicht wird.

Bei Spaltlampen wie sie beispielsweise in [1] beschrieben sind, werden zur Erzeugung von Spaltabbildungen überwiegend mechanisch/optische Elemente, wie Spaltblenden benutzt. Die für eine hohe optische Detailauflösung innerhalb des optischen Schnittes erforderlichen variablen und möglichst geringen Spaltbreiten sind sehr schwer realisierbar. Außerdem ist die Justierung der mechanischen Baugruppen sehr aufwendig, was durch die Wärmeausdehnung der Baugruppen noch erschwert wird. Eine Reproduzierbarkeit exakter Spaltbreiten ist kaum möglich. Da es sich bei der Spaltbildprojektion um eine optische Abbildung mit physikalisch begrenzter Schärfentiefe handelt, muss die Abbildung immer streng auf den Ort der Untersuchung fokussiert werden. Ein in der gesamten Ausdehnung des menschlichen Auges scharfes Schnittbündel lässt sich mit den bisher genannten Lösungen nicht erzielen.

In der DE 198 12 050 A1 sind ein Verfahren und eine Anordnung zur Beleuchtung bei einem Augenmikroskop beschrieben. Die verschiedensten Leuchtmarkengeometrien werden mit Hilfe opto-elektronischer Bauelemente erzeugt. Die Leuchtfeldgeometrien werden dabei auf den Augenvorder- oder Hintergrund projiziert und dienen der allgemeinen Untersuchung des Auges.

Die DE 199 43 735 A1 beschreibt ein Verfahren und eine Vorrichtung zur gezielten Bestrahlung eines Auges mittels Licht aus dem sichtbaren und/oder nahinfraroten Wellenlängenbereich. Durch die Bestrahlung werden irreversible chemische Veränderungen der Augenlinsen-Substanz hervorgerufen, die eine Veränderung des Brechungsindex und/oder der Transmissionseigenschaften für die sichtbare Nutzstrahlung zur Folge haben und dadurch ein fehlerreduziertes Sehen ermöglichen. Die erfolgreiche Behandlung setzt dabei eine möglichst engmaschige und vollflächige Bestimmung der Verteilung der Brechkraft des zu behandelnden Auges voraus. Aus diesen Werten werden die nach der Behandlung gewünschte Brechkraftverteilung und die dafür erforderlichen Daten der Bestrahlung ermittelt. Als nachteilig wirkt sich bei dieser Lösung aus, dass die Bestrahlung in der Regel nur punktweise nacheinander erfolgen kann und das Behandlungsverfahren dadurch zeitintensiv ist. Für die Dauer der Behandlung ist deshalb eine Fixierung des Augapfels unerlässlich.

In den Patentschriften US 5,404,884; US 5,139,022 und US 6,275,718 sind Verfahren und Anordnungen zur Beleuchtung der vorderen Augensegmente beschrieben, bei denen als Lichtquelle ein planar konfigurierter Laser verwendet wird. Nachteilig bei diesen Lösungen ist die eingeschränkte Variabilität der Leuchtfeldgeometrien. Weiterhin hat das Aufnahmesystem für das Streulicht vom Auge eine physikalisch begrenzte Schärfentiefe, die den Ausdehnungsbereich des scharfen Laser-Schnittbildes nicht vollständig erfassen kann.

### Literatur:

[1] Rassow, B. u. a., "Ophthalmologisch-optische Instrumente", 1987, Ferdinand Enke Verlag Stuttgart, S. 99 ff und 137 ff

Bei den derzeitigen ophthalmologischen Geräten wirkt sich die gerade oder sogar entgegengesetzt gekrümmte Bildebene der Beleuchtungs- bzw. Bestrahlungskomponenten als nachteilig aus. Dadurch weisen die in oder auf das Auge projezierten Strukturen nur in der Mitte des Bildfeldes, an der Sehachse die erforderliche Bildschärfe auf. In den Außen- und Randbereichen fächern die feinen Strukturen auf, werden unscharf und verlieren deutlich an Intensität. Eine Auswertung der Verzerrung der Strukturen ist damit erschwert bzw. überhaupt nur in einem begrenzten Bereich möglich.

Der vorliegenden Erfindung liegt die Aufgabe zugrunde die Bildfelder der bekannten Beleuchtungs- bzw. Bestrahlungskomponenten ophthalmologischer Diagnose- und Therapiegeräten dahingehend zu verbessern, dass in oder auf das Auge projizierte Strukturen, Bilder und Zeichen eine gleichmäßig hohe Abbildungsqualität über weite Bereiche des Auges aufweisen.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Mit der vorgestellten Lösung läßt sich eine gleichmäßige Abbildungsqualität über weite Bereiche des Auges erzielen. Damit sind bei entsprechenden Auswertealgorithmen schnellere und umfassendere Aussagen möglich. Insbesondere ist die Lösung zur Bestimmung der biometrischen Daten eines Auges anwendbar, bei der großflächige gekrümmte Augenareale mit einer sehr feinen Struktur beleuchtet werden.

Die Erfindung wird nachfolgend anhand eines Ausführungsbeispieles beschrieben. Dazu zeigen
- Figur 1:: einen schematischen Beleuchtungsstrahlenganges mit einem diffraktiven optischen Element (DOE) und die
- Figuren 2 und 3:: an das Auge angepasste, gekrümmte Bildebenen.

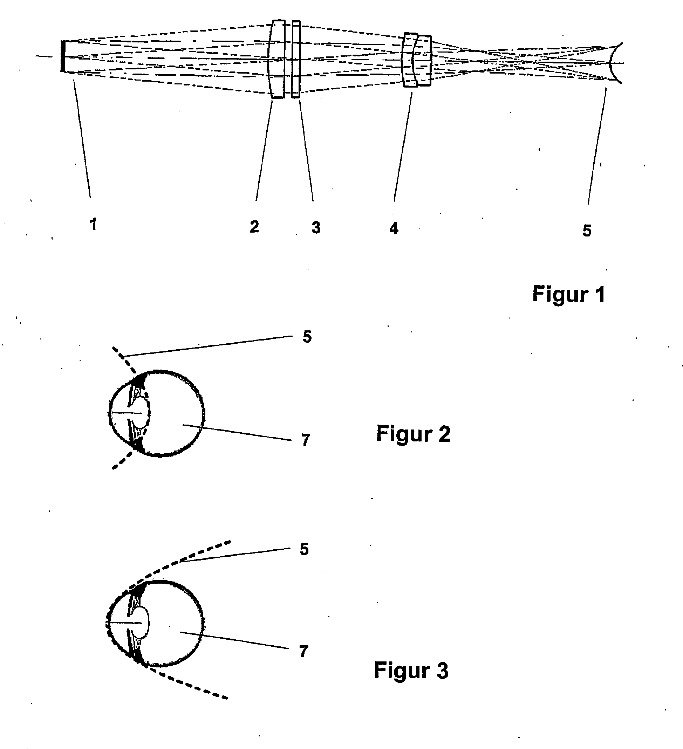

Bei der Anordnung zur Bildfeldverbesserung in ophthalmologischen Geräten wird im Beleuchtungsstrahlengang der Bestrahlungseinheit ein diffraktives optisches Element (DOE) **3** angeordnet, um eine gezielte Formung der Bildebene **5** zu erreichen. Das diffraktive optische Element **3** kann sich dabei auf der Oberfläche eines anderen optischen Elementes befinden oder, wie in **Figur 1** dargestellt, als separates Element im Strahlengang eingeordnet sein. Dabei ist sowohl die Art der verwendeten Lichtquelle als auch die Art der Strahlformung, d. h. der Struktur- oder Mustererzeugung unerheblich (nicht dargestellt). **Figur 1** zeigt deshalb den Strahlenverlauf ausgehend vom jeweiligen Beleuchtungsmuster **1.** Die Beleuchtungsstrahlen verlaufen, ausgehend vom Beleuchtungsmuster **1,** über eine als erstes Abbildungssystem dienenden Optik **2** zum DOE **3.** Durch das DOE **3** wird der Strahlenverlauf der Beleuchtungsstrahlen derart verändert, dass im zu bestrahlenden Auge **7** eine, der Krümmung des jeweils zu bestrahlenden Elementes, angepasste Bildebene **5** entsteht. Dazu zeigt **Figur 2** eine an die Rückfläche der Augenlinse und **Figur 3** eine an die Vorderfläche der Hornhaut angepasste Bildebene **5**.

Durch die erfindungsgemäße Anordnung im Beleuchtungsstrahlengang ophthalmologischer Geräte wird eine sphärische, der Krümmung des Auges **7** angepassten, Bildebene **5** erzeugt, damit die projizierten Zeichen oder Strukturen eine gleichmäßig hohe Abbildungsqualität über weite Bereiche, ausgehend von der optischen Achse bis in die Randbereiche des Auges **7** aufweisen.

In einer weiteren technischen Ausgestaltung ist vorgesehen das diffraktive optische Element **3** schwenkbar zu gestalten. Dadurch kann die Wirkung gezielt ein- und ausgeschaltet werden. Das diffraktive optische Element **3** ist dabei in Verbindung mit dem Gesamtsystem für eine definierte Krümmung der Hornhaut optimiert. Für andere Hornhautkrümmungen sind dementsprechend andere diffraktive optische Elemente **3** erforderlich, die wiederum in Verbindung mit dem Gesamtsystem für diese Krümmung der Hornhaut optimiert sind.

Da verschiedene DOE **3** bezüglich der Wellenlänge sehr empfindlich reagieren, ist es vorteilhaft dies mit entsprechenden Farbfiltern zu kombinieren. Dadurch kann die Wellenlänge für das DOE **3** optimal auf die Therapiewellenlänge abgestimmt werden. Für unterschiedliche Anwendungsfälle kann somit das jeweils optimale DOE **3** benutzt und mit dem entsprechenden Filter kombiniert werden. Die Filter als auch die DOE **3** sind dazu vorteilhafterweise auf ein oder mehreren Wechslern, wie beispielsweise Schiebern oder Rädern angeordnet. Wahlweise können dabei sowohl die DOE **3** als auch die Filter einzeln verwendet oder miteinander kombiniert werden.

Die Anordnung zur Bildfeldverbesserung kann zusätzlich über eine variable, einstellbare, numerische Apertur verfügen, mit der einerseits die Intensität des Beleuchtungsmuster **1** in der Bildebene **5** geregelt und andererseits auch die Strahldichte im zu bestrahlenden Auge **7** beeinflusst werden kann, um die gültigen Grenzwerte für die Strahlendosis einzuhalten. Die Beeinflussung der Apertur kann im einfachsten Fall über eine variable Aperturblende im Beleuchtungsstrahlengang erfolgen.

Die Anordnung zur Bildfeldverbesserung kann weiterhin eine einstellbare Brenn- oder Schnittweite besitzen. Damit kann die Bildlage im Zielgebiet entlang der optischen Achse definiert verschoben werden. Beispielsweise kann eine scharfe Abbildung mit hoher Apertur speziell auf die Linsenvorderfläche bzw. Linsenrückfläche gelegt werden. Auch Zwischenstellungen sind bei Bedarf frei wählbar. Diese Verschiebemöglichkeit kann vorteilhafter Weise mit einer Abstandskontrolle und einer Fokussierhilfe kombiniert werden. Damit kann die Position auch entlang der optischen Achse genau eingestellt und konstant gehalten werden. Die Fokussierhilfe kann auf dem Prinzip der Mehrfach-Spotabbildung unter hoher Apertur erfolgen, so daß alle einzelnen Spots nur in der Zielebene zusammenfallen und einen einzelnen Spot ergeben. Die Realisierung einer Abstandskontrolle kann beispielsweise über bekannte Vierquadrantenempfänger erfolgen, die den Scheitelreflex der Hornhaut auswerten.

Die Funktion der verstellbaren Aperturblende kann mit der Funktion der verstellbaren Schnitt- oder Brennweite und der Realisierung dynamischer Mustervorteilhaft kombiniert werden, um gezielt Bestrahlungsabfolgen mit speziellen Mustern an bestimmten Orten zu applizieren, ohne die entsprechenden Grenzwerte zu überschreiten. Alle Bestrahlungsparameter können aufgezeichnet und abgespeichert werden. Die Positionskontrolle und - korrektur kann mittels einer Eye-Tracker-Einheit erfolgen und sichert eine exakte Bestrahlung nur im ausgerichteten Zustand.

Aus der durch die erfindungsgemäße Anordnung erzeugten sphärisch gekrümmte und an das zu bestrahlende Auge **7** angepassten Bildebene **5** resultiert über den Beobachtungsstrahlengang eine zwangsläufig ebenfalls gekrümmte Abbildungsebene im Auge des Betrachters und/oder einer Einheit zum Dokumentieren/Archivieren. Durch die zusätzlich Anordnung ein oder mehrere diffraktiver optischer Elemente im Beobachtungsstrahlengang läßt sich wieder eine ebene Abbildungsebene erzeugen. Diese ist beispielsweise für die fotografische Dokumentation der durchgeführten Diagnose und Therapie sowie deren Ergebnisse erforderlich. Dadurch kann eine gleichmäßig hohe Abbildungsqualität über die gesamte ebene Abbildungsebene erzeugt werden.

Da die diffraktiven optischen Elemente bezüglich der Wellenlänge sehr empfindlich reagieren, ist es auch hier vorteilhaft diese mit entsprechenden Farbfiltern zu kombinieren. Dabei sollte die Wellenlänge für die diffraktiven optischen Elemente optimal auf eine Beobachtungswellenlänge abgestimmt werden, um die Augen des Betrachters vor der Therapie-/Diagnosestrahlung zu schützen.

Die diffraktiven optischen Elemente können dabei auf der Oberfläche anderer optischer Elemente oder als separate Elemente im Strahlengang angeordnet sein. Die diffraktiven optischen Elementen im Beobachtungsstrahlengang können analog denen im Beleuchtungsstrahlengang schwenkbar und in ihrer Wellenlänge durch Filter veränderbar sein. Sowohl die Filter als auch die diffraktiven optischen Elemente können auf ein oder mehreren Wechslern, wie beispielsweise Schiebern oder Rädern angeordnet werden. Wahlweise können dabei sowohl die diffraktiven optischen Elemente als auch die Filter einzeln verwendet oder miteinander kombiniert werden.

Durch Einsatz diffraktiver optischer Elemente **3** im Beleuchtungsstrahlengang können optische Wirkungen erzielt werden, die mit klassischen optischen Bauelementen wie Achromaten und Linsen gar nicht oder nur mit wesentlich größerem Aufwand erreicht werden können. Dabei kann die Bildebene der spärischen Kontur des Auges angepasst werden, so dass die auf oder in das Auge projizierten Zeichen und Strukturen eine gleichmäßig hohe Abbildungsqualität sowohl im Zentrum als auch im Randbereich aufweisen.

Dies ist insbesondere dann von Bedeutung, wenn Gitter oder Spaltstrukturen zu Messzwecken großflächig auf die Augenoberfläche projiziert werden, um durch eine anschließende Triangulation die Biometriedaten zu bestimmen.

Die vorliegende Erfindung ist aber auch zur Erzeugung einer variablen Beleuchtung für die Diagnose und Therapie am menschlichen Auge, insbesondere zur Bestrahlung der Augenlinse und anderer Augenabschnitte wie Kornea oder Retina anwendbar. Dazu kann die Anordnung zur Bildfeldverbesserung in verschiedensten ophthalmologischen Geräte, wie Funduskameras, Spaltlampen, Laserscanner, OPMI-Geräte oder auch Operationsmikroskopen eingesetzt werden.

Selbst für die Bestrahlung einer in ein Auge eingebrachten Linse oder anderer optisch wirksamen Hilfsmittel ist die Anordnung einsetzbar. Bei IOL aus Kunststoff, gemäß WO 00/41650 und/oder WO 01/71411 werden durch Bestrahlung der Linse Polymerisationsvorgänge angeregt, die irreversible chemische Veränderungen der Linsen-Substanz zur Folge haben. Durch diese Vorgänge können der Brechungsindex und/oder das Transmissionsverhalten für die sichtbare Nutzstrahlung bzw. die geometrische Form der IOL definiert verändert und dadurch ein fehlerreduziertes Sehen ermöglicht werden.

## Patentansprüche

1. Anordnung zur Bildfeldverbesserung bei ophthalmologischen Geräten, bei der im Beleuchtungsstrahlengang zusätzlich ein oder mehrere diffraktive optische Elemente (3) zur gezielten Formung der Bildebene (5) angeordnet sind.

2. Anordnung zur Bildfeldverbesserung nach Anspruch 1, bei der sich das oder die diffraktiven optischen Elemente (3) auf der Oberfläche anderer optischer Elemente befinden oder als separate Elemente im Beleuchtungsstrahlengang angeordnet sind.

3. Anordnung zur Bildfeldverbesserung nach mindestens einem der vorgenannten Ansprüche, bei der das oder die diffraktiven optischen Elemente (3) in den Beleuchtungsstrahlengang ein- und ausgeschwenkt werden können.

4. Anordnung zur Bildfeldverbesserung nach mindestens einem der vorgenannten Ansprüche, bei der die Wellenlänge für das oder die diffraktiven optischen Elemente (3) durch Filter veränderbar ist/sind.

5. Anordnung zur Bildfeldverbesserung nach mindestens einem der vorgenannten Ansprüche, bei der die diffraktiven optischen Elemente (3) zur Formung verschiedener Bildebenen (5) wahlweise in den Beleuchtungsstrahlengang ein- und ausgeschwenkt werden können.

6. Anordnung zur Bildfeldverbesserung nach mindestens einem der vorgenannten Ansprüche, bei der die verschiedenen diffraktiven optischen Elemente (3) auf ein oder mehreren Wechslern angeordnet sind, wobei die diffraktiven optischen Elemente (3) einzeln oder miteinander kombiniert in den Beleuchtungsstrahlengang eingeschwenkt werden können.

7. Anordnung zur Bildfeldverbesserung nach mindestens einem der vorgenannten Ansprüche, bei der verschiedene Filter auf ein oder mehreren Wechslern angeordnet sind, wobei die Filter einzeln oder miteinander kombiniert in den Beleuchtungsstrahlengang eingeschwenkt werden können.

8. Anordnung zur Bildfeldverbesserung nach mindestens einem der vorgenannten Ansprüche, bei der das vorhandene optische Abbildungssystem (4) über eine einstellbare numerische Apertur und eine variable Schnittweite verfügt.

9. Anordnung zur Bildfeldverbesserung nach mindestens einem der vorgenannten Ansprüche, bei der eine zusätzliche Einheit zur Abstandskontrolle und als Fokussierhilfe vorgesehen ist.

10. Anordnung zur Bildfeldverbesserung nach mindestens einem der vorgenannte Ansprüche, bei der eine Eye-Tracker-Einheit zur Positionskontrolle und -korrektur vorgesehen ist.

11. Anordnung zur Bildfeldverbesserung bei ophthalmologischen Geräten, bei der im Beobachtungsstrahlengang zusätzlich ein oder mehrere diffraktive optische Elemente zur gezielten Formung der Abbildungsebene angeordnet sind.

12. Anordnung zur Bildfeldverbesserung nach Anspruch 11, bei der sich das oder die diffraktiven optischen Elemente - auf der Oberfläche anderer optischer Elemente befinden oder als separate Elemente im Beobachtungsstrahlengang angeordnet sind.

13. Anordnung zur Bildfeldverbesserung nach mindestens einem der Ansprüche 11 und 12, bei der das oder die diffraktiven optischen Elemente in den Beobachtungsstrahlengang ein- und ausgeschwenkt werden können.

14. Anordnung zur Bildfeldverbesserung nach mindestens einem der vorgenannten Ansprüche, bei der die Wellenlänge für das oder die diffraktiven optischen Elemente durch Filter veränderbar ist/sind.

15. Anordnung zur Bildfeldverbesserung nach mindestens einem der vorgenannten Ansprüche, bei der die diffraktiven optischen Elemente zur Formung verschiedener Abbildungsebenen wahlweise in den Beobachtungsstrahlengang ein- und ausgeschwenkt werden können.

16. Anordnung zur Bildfeldverbesserung nach mindestens einem der vorgenannten Ansprüche, bei der die verschiedenen diffraktiven optischen Elemente auf ein oder mehreren Wechslern angeordnet sind, wobei die diffraktiven optischen Elemente einzeln oder miteinander kombiniert in den Beobachtungsstrahlengang eingeschwenkt werden können.

17. Anordnung zur Bildfeldverbesserung nach mindestens einem der vorgenannten Ansprüche, bei der verschiedene Filter auf ein oder mehreren Wechslern angeordnet sind, wobei die Filter einzeln oder miteinander kombiniert in den Beobachtungsstrahlengang eingeschwenkt werden können.

## Claims

1. Arrangement for improving the image field in ophthalmological appliances, in which one or more diffractive optical elements (3) are initially arranged in the illumination beam path for the purpose of specifically shaping the image plane (5).

2. Arrangement for improving the image field according to Claim 1, in which the diffractive optical element(s) (3) is/are located on the surface of other optical elements, or is/are arranged as separate elements in the illumination beam path.

3. Arrangement for improving the image field according to at least one of the abovementioned claims, in which the diffractive optical element(s) (3) can be pivoted into and out of the illumination beam path.

4. Arrangement for improving the image field according to at least one of the abovementioned claims, in which the wavelength for the diffractive optical element(s) (3) can be varied by filters.

5. Arrangement for improving the image field according to at least one of the abovementioned claims, in which the diffractive optical elements (3) can optionally be pivoted into and out of the illumination beam path for the purpose of shaping various image planes (5).

6. Arrangement for improving the image field according to one or more of the abovementioned claims, in which the various diffractive optical elements (3) are arranged on one or more changers, it being possible for the diffractive optical elements (3) to be pivoted into the illumination beam path individually or in a fashion combined with one another.

7. Arrangement for improving the image field according to at least one of the abovementioned claims, in which various filters are arranged on one or more changers, it being possible for the filters to be pivoted into the illumination beam path individually or in a fashion combined with one another.

8. Arrangement for improving the image field according to at least one of the abovementioned claims, in which the available optical imaging system (4) has an adjustable numerical aperture and a variable back focus.

9. Arrangement for improving the image field according to at least one of the abovementioned claims, in which an additional unit is provided for distance monitoring and as a focusing aid.

10. Arrangement for improving the image field according to at least one of the abovementioned claims, in which an eye tracker unit is provided for position monitoring and position correction.

11. Arrangement for improving the image field in case of ophthalmological appliances, in which one or more diffractive optical elements are additionally arranged in the observation beam path for the purpose of specifically shaping the imaging plane.

12. Arrangement for improving the image field according to Claim 11, in which the diffractive optical element(s) is/are located on the surface of other optical elements, or is/are arranged as separate elements in the observation beam path.

13. Arrangement for improving the image field according to at least one of Claims 11 and 12, in which the diffractive optical element(s) can be pivoted into and out of the observation beam path.

14. Arrangement for improving the image field according to at least one of the abovementioned claims, in which the wavelength for the diffractive optical element(s) can be varied by filters.

15. Arrangement for improving the image field according to at least one of the abovementioned claims, in which the diffractive optical elements can optionally be pivoted into and out of the observation beam path for the purpose of shaping various image planes.

16. Arrangement for improving the image field according to one or more of the abovementioned claims, in which the various diffractive optical elements are arranged on one or more changers, it being possible for the diffractive optical elements to be pivoted into the observation beam path individually or in a fashion combined with one another.

17. Arrangement for improving the image field according to at least one of the abovementioned claims, in which various filters are arranged on one or more changes, it being possible for the filters to be pivoted into the observation beam path individually or in a fashion combined with one another.

## Revendications

1. Ensemble d'amélioration du champ d'image d'appareils ophtalmologiques, dans lequel un ou plusieurs éléments optiques diffractants (3) sont de plus disposés dans le parcours des rayons d'éclairage pour former le plan d'image (5) de manière contrôlée.

2. Agencement d'amélioration du champ d'image selon la revendication 1, dans lequel le ou les éléments optiques diffractants (3) sont situés à la surface d'autres éléments optiques ou sont disposés comme éléments distincts dans le parcours des rayons d'éclairage.

3. Agencement d'amélioration du champ d'image selon au moins l'une des revendications précédentes, dans lequel le ou les éléments optiques diffractants (3) peuvent être insérés dans le parcours des rayons d'éclairage ou en être extraits.

4. Agencement d'amélioration du champ d'image selon au moins l'une des revendications précédentes, dans lequel la longueur d'onde du ou des éléments optiques diffractants (3) peut être modifiée par des filtres.

5. Agencement d'amélioration du champ d'image selon au moins l'une des revendications précédentes, dans lequel les éléments optiques diffractants (3) peuvent sélectivement être insérés dans le parcours des rayons d'éclairage pour former différents plans d'image (5) ou en être extraits.

6. Agencement d'amélioration du champ d'image selon au moins l'une des revendications précédentes, dans lequel les différents éléments optiques diffractants (3) sont disposés sur un ou plusieurs changeurs, les éléments optiques diffractants (3) pouvant être insérés séparément ou en combinaison de plusieurs dans le parcours des rayons d'éclairage.

7. Agencement d'amélioration du champ d'image selon au moins l'une des revendications précédentes, dans lequel différents filtres sont disposés sur un ou plusieurs changeurs, les filtres pouvant être insérés séparément ou en combinaison de plusieurs dans le parcours de rayons d'éclairage.

8. Agencement d'amélioration du champ d'image selon au moins l'une des revendications précédentes, dans lequel le système optique (4) de formation d'image dispose d'une ouverture numérique réglable et d'une distance focale variable.

9. Agencement d'amélioration du champ d'image selon au moins l'une des revendications précédentes, dans lequel une unité supplémentaire de contrôle de distance est prévue comme accessoire de focalisation.

10. Agencement d'amélioration du champ d'image selon au moins l'une des revendications précédentes, dans lequel une unité de suivi de l'oeil est prévue pour le contrôle et la correction de la position.

11. Agencement d'amélioration du champ d'image d'appareils ophtalmologiques, dans lequel un ou plusieurs éléments optiques diffractants sont de plus disposés dans le parcours des rayons d'éclairage pour former de manière contrôlée le plan de formation d'image.

12. Agencement d'amélioration du champ d'image selon au moins l'une des revendications précédentes selon la revendication 11, dans lequel le ou les éléments optiques diffractants sont situés sur la surface d'autres éléments optiques ou sont disposés comme éléments distincts dans le parcours des rayons d'observation.

13. Agencement d'amélioration du champ d'image selon au moins l'une des revendications 11 et 12, dans lequel le ou les éléments optiques diffractants peuvent être insérés dans le parcours des rayons d'observation et en être extraits.

14. Agencement d'amélioration du champ d'image selon au moins l'une des revendications précédentes, dans lequel la longueur d'onde du ou des éléments optiques diffractants peut être modifiée par des filtres.

15. Agencement d'amélioration du champ d'image selon au moins l'une des revendications précédentes, dans lequel les éléments optiques diffractants peuvent sélectivement être insérés dans le parcours des rayons d'observation pour former différents plans de formation d'image ou en être extraits.

16. Agencement d'amélioration du champ d'image selon au moins l'une des revendications précédentes, dans lequel les différents éléments optiques diffractants sont disposés sur un ou plusieurs changeurs, les éléments optiques diffractants pouvant être insérés séparément ou en combinaison de plusieurs dans le parcours des rayons d'observation.

17. Agencement d'amélioration du champ d'image selon au moins l'une des revendications précédentes, dans lequel différents filtres sont disposés sur un ou plusieurs changeurs, les filtres pouvant être insérés dans le parcours des rayons d'observation séparément ou en combinaison de plusieurs.
